# EUROPEAN PATENT APPLICATION

(11) **EP 3 323 370 A2**
(43) Date of publication of application: **23.05.2018**
(21) Application number: 17202906.8
(22) Date of filing: 21.11.2017
(51) Int. Cl.: A61B 34/20

(54) **ELECTROMAGNETIC NAVIGATION REGISTRATION USING ULTRASOUND**

(30) Priority: 21.11.2016 US 201662424853 P; 16.11.2017 US 201715815262
(71) Applicant: Covidien LP, Mansfield, MA 02048 (US)
(72) Inventor: KOYRAKH, Lev A., Plymouth, MN Minnesota 55442 (US); STOPEK, Joshua B., Minneapolis, MN Minnesota 55419 (US)
(74) Representative: Maschio, Antonio

(57) **Abstract**

A method for electromagnetic navigation registration is provided. The method includes storing, in a memory, a mapping that associates electromagnetic field-based signal values with corresponding locations within a three-dimensional model of a luminal network. An ultrasound signal is received from an ultrasound probe. Based on the ultrasound signal, an ultrasound-based location of a target in a patient relative to the three-dimensional model is determined. At least a portion of the mapping is updated based on the ultrasound-based location of the target.

## Description

### CROSS REFERENCE TO RELATED APPLICATION

The present application claims the benefit of and priority to U.S. Provisional Application Serial No. 62/424,853, filed on November 21, 2016 the entire contents of which are incorporated herein by reference.

### BACKGROUND

### Technical Field

The present disclosure generally relates to electromagnetic navigation and imaging in patients, and more particularly, to a method for electromagnetic navigation registration using ultrasound.

### Background of Related Art

A bronchoscope is commonly used to inspect the airway of a patient. Typically, the bronchoscope is inserted into a patient's airway through the patient's nose or mouth or another opening, and can extend into the lungs of the patient. The bronchoscope typically includes an elongated flexible tube having an illumination assembly for illuminating the region distal to the bronchoscope's tip, an imaging assembly for providing a video image from the bronchoscope's tip, and a working channel through which an instrument, such as a diagnostic instrument (for example, a biopsy tool), a therapeutic instrument, and/or another type of tool, can be inserted.

Electromagnetic navigation (EMN) systems and methods have been developed that utilize a three-dimensional model (or an airway tree) of the airway, which is generated from a series of computed tomography (CT) images generated during a planning stage. One such system has been developed as part of Medtronic Inc.'s ILOGIC® ELECTROMAGNETIC NAVIGATION BRONCHOSCOPY® (ENB™) system. The details of such a system are described in U.S. Patent No. 7,233,820, entitled ENDOSCOPE STRUCTURES AND TECHNIQUES FOR NAVIGATING TO A TARGET IN BRANCHED STRUCTURE, filed on April 16, 2003, the entire contents of which are hereby incorporated herein by reference. Additional aspects of such a system relating to image registration and navigation are described in U.S. Patent No. 8,218,846, entitled AUTOMATIC PATHWAY AND WAYPOINT GENERATION AND NAVIGATION METHOD, filed on May 14, 2009; U.S. Patent Application Publication No. 2016/0000356, entitled REAL-TIME AUTOMATIC REGISTRATION FEEDBACK, filed on July 2, 2015; and U.S. Patent Application Publication No. 2016/0000302, entitled SYSTEM AND METHOD FOR NAVIGATING WITHIN THE LUNG, filed on June 29, 2015; the entire contents of each of which are hereby incorporated herein by reference.

Such EMN systems and methods typically involve registering spatial locations of an electromagnetic sensor to corresponding spatial locations in the airway tree. To perform the registration, a lung survey is performed by collecting (or sampling) signal values from the electromagnetic sensor at different portions of the airway, and generating a point cloud that is utilized to map an electromagnetic field-based coordinate system to a coordinate system of the airway tree and/or of the CT scan itself.

In some cases, a bronchoscope may be too large to reach beyond the few first generations of airway branches, and may therefore be unable to sample signal values within or near branches close to peripheral targets at which some ENB procedures are aimed. Thus, the point cloud generated during some lung surveys may be somewhat limited. Also, because the lungs are flexible, there may be differences between the structure of the airways at the time the CT scan was generated and the structure of the airways during a subsequent EMN procedure. Together these factors may cause CT-to-body divergence, which may result in registration errors and lead to errors in locating ENB targets.

Given the foregoing, it would be beneficial to have improved EMN registration systems and methods that are capable of updating a registration within or near peripheral airways and/or at a location of a target itself.

### SUMMARY

In accordance with an aspect of the present disclosure, a method for electromagnetic navigation registration is provided. The method includes storing, in a memory, a mapping that associates electromagnetic field-based signal values with corresponding locations within a three-dimensional model of a luminal network. An ultrasound signal is received from an ultrasound probe. Based on the ultrasound signal, an ultrasound-based location of a target in a patient relative to the three-dimensional model is determined. At least a portion of the mapping is updated based on the ultrasound-based location of the target.

In another aspect of the present disclosure, the method further includes receiving an electromagnetic sensor signal from an electromagnetic sensor. Based on a value of the electromagnetic sensor signal and the mapping, an electromagnetic sensor location within the three-dimensional model that corresponds to the value of the electromagnetic sensor signal is identified. An ultrasound probe location within the three-dimensional model that corresponds to the ultrasound signal is identified, based on the electromagnetic sensor location and a spatial relationship between the ultrasound probe and the electromagnetic sensor.

In yet another aspect of the present disclosure, the method further includes determining, based on the ultrasound signal, a location of the target relative to the ultrasound probe. The ultrasound-based location of the target is determined based on (i) the location of the target relative to the ultrasound probe and (ii) the electromagnetic sensor location and/or the ultrasound probe location.

In a further aspect of the present disclosure, the spatial relationship between the ultrasound probe and the electromagnetic sensor is fixed.

In still another aspect of the present disclosure, the spatial relationship between the ultrasound probe and the electromagnetic sensor is variable.

In another aspect of the present disclosure, the receiving of the ultrasound signal occurs while the ultrasound probe and the electromagnetic sensor are positioned in respective locations in the patient, and the receiving of the electromagnetic sensor signal occurs while the ultrasound probe and the electromagnetic sensor are positioned in those same respective locations in the patient.

In yet another aspect of the present disclosure, the locations within the three-dimensional model include a modeled location of the target, and the mapping associates one or more of the electromagnetic field based signal values with the modeled location of the target. The method further includes determining a difference between the modeled location of the target and the ultrasound-based location of the target, based on the ultrasound probe location and/or the electromagnetic sensor location.

In a further aspect of the present disclosure, the method also includes displaying, via a graphical user interface: (i) at least a portion of the three-dimensional model, based on the electromagnetic sensor location and/or the ultrasound probe location, (ii) an indication of the modeled location of the target relative to at least the portion of the three-dimensional model, and (iii) an indication of the ultrasound-based location of the target relative to at least the portion of the three-dimensional model.

In still another aspect of the present disclosure, the method further includes generating an image of the target based on the ultrasound signal, with the indication of the ultrasound-based location of the target being the image of the target.

In another aspect of the present disclosure, the displaying includes simultaneously displaying a combined view of: (i) the indication of the modeled location of the target relative to at least the portion of the three-dimensional model, and (ii) the indication of the ultrasound-based location of the target relative to at least the portion of the three-dimensional model.

In yet another aspect of the present disclosure, the locations within the three-dimensional model include a modeled location of the target, and the mapping associates one or more of the electromagnetic field-based signal values with the modeled location of the target. The method further includes determining a difference between the modeled location of the target and the ultrasound-based location of the target, based on image processing of the combined view of the indication of the modeled location of the target and the indication of the ultrasound-based location of the target.

In a further aspect of the present disclosure, the updating at least the portion of the mapping is automatically performed based on the difference between the modeled location of the target and the ultrasound-based location of the target.

In still another aspect of the present disclosure, the method further includes receiving, by way of a user interface, an indication of a location within at least the displayed portion of the three-dimensional model that corresponds to the target. The determining of the ultrasound-based location of the target is based on the indication of the location that corresponds to the target.

In another aspect of the present disclosure, the method further includes receiving, by way of the user interface, a command to update the mapping, and the updating at least the portion of the mapping is performed in response to the receiving of the command.

In yet another aspect of the present disclosure, the locations within the three-dimensional model include a modeled location of the target, and the mapping associates one or more of the electromagnetic-field based signal values with the modeled location of the target. The method further includes determining a difference between the modeled location of the target and the ultrasound-based location of the target.

In a further aspect of the present disclosure, the updating at least the portion of the mapping is based on the difference between the modeled location of the target and the ultrasound-based location of the target.

In still another aspect of the present disclosure, the updating at least the portion of the mapping includes modifying the mapping to associate a different one or more of the electromagnetic field based signal values with the modeled location of the target.

In another aspect of the present disclosure, the method further includes executing an interpolation algorithm based on the difference between the modeled location of the target and the ultrasound-based location of the target. The updating at least the portion of the mapping further includes modifying the mapping to associate a plurality of the electromagnetic-field based signal values with a plurality of the locations within the three-dimensional model, respectively, based on a result of the executing of the interpolation algorithm.

In yet another aspect of the present disclosure, the luminal network is an airway of the patient.

In accordance with another aspect of the present disclosure, another method for electromagnetic navigation registration is provided. The method includes receiving a signal from an ultrasound probe. Based on the signal received from the ultrasound probe, an ultrasound image of a target in a patient is generated. Based on the ultrasound image, a location of the target relative to the ultrasound probe is determined. A signal is received from an electromagnetic sensor. Based on the signal received from the electromagnetic sensor, a location of the electromagnetic sensor relative to a three-dimensional model of a luminal network is determined. An ultrasound-based location of the target relative to the three-dimensional model is determined, based on the location of the target relative to the ultrasound probe, the location of the electromagnetic sensor relative to the three-dimensional model, and a spatial relationship between the ultrasound probe and the electromagnetic sensor. Based on the ultrasound-based location of the target, a mapping that associates electromagnetic field-based signal values with corresponding locations within the three-dimensional model is updated.

Any of the above aspects and embodiments of the present disclosure may be combined without departing from the scope of the present disclosure.

### BRIEF DESCRIPTION OF THE DRAWINGS

Various embodiments of the present disclosure are described herein with reference to the drawings wherein:
FIG. 1 is a schematic illustration of an example electromagnetic navigation (EMN) system and two example catheter guide assemblies, of which one or both may be used within the EMN system, in accordance with various embodiments of the present disclosure;
FIG. 2 is a perspective view of an example catheter guide assembly of the EMN system of FIG. 1, in accordance with the present disclosure;
FIG. 2A is an enlarged view of an example embodiment of a distal portion of the catheter guide assembly of FIG. 2 indicated by area "A";
FIG. 2B is an enlarged view of an alternative example embodiment of the distal portion of the catheter guide assembly of FIG. 2 indicated by area "A";
FIG. 3 is a flow diagram illustrating an example method for electromagnetic navigation registration, in accordance with an embodiment of the present disclosure;
FIG. 4A is an illustration of an example collection of survey points forming part of a Body-Space model of a patient's airway;
FIG. 4B is an illustration of an example collection of reference points forming part of a three-dimensional model of a patient's airway;
FIG. 5A is an illustration of an example user interface of the workstation of FIG. 1 presenting a view for performing and updating registration in accordance with the present disclosure;
FIG. 5B is an illustration of an example user interface of the workstation of FIG. 1 presenting a view for performing and updating registration in accordance with the present disclosure; and
FIG. 6 is a schematic of example components of a workstation that may be implemented in the EMN system of FIG. 1, in accordance with an embodiment of the present disclosure.

### DETAILED DESCRIPTION

The present disclosure is directed to devices, systems, and methods for updating a registration of a three-dimensional luminal network model (for example, a bronchial tree model) (also referred to herein as a "three-dimensional model") with a patient's airway. In particular, the present disclosure relates to using an ultrasound probe to acquire one or more additional reference points to update a previous registration of a three-dimensional model with a patient's airway. The location of a target identified using an ultrasound probe (also referred to herein as an ultrasound-based location of the target) can be compared to a corresponding modeled target location within the three-dimensional model. If the two locations differ, the registration of the three-dimensional model with the patient's airway can be updated accordingly, for instance, by correcting the modeled target location based on the ultrasound-based target location. The term "target," as used herein, generally refers to any location of interest within a patient. For example, the target may be a target of biopsy, treatment, or assessment, or a particular portion of the patient's lungs, such as a location corresponding to a fiducial point or a location where an airway branches, or any other location within or outside of a luminal network of the patient.

Various methods for generating the three-dimensional model and identifying a target are envisioned, some of which are more fully described in U.S. Patent Application Publication Nos. 2014/0281961, 2014/0270441, and 2014/0282216, all entitled PATHWAY PLANNING SYSTEM AND METHOD, filed on March 15, 2013, the entire contents of all of which are incorporated herein by reference. Following generation of the three-dimensional model and identification of the target, the three-dimensional model is registered with the patient's airway. Various methods of manual and automatic registration are envisioned, some of which are more fully described in U.S. Patent Application Publication No. 2016/0000356.

To further improve registration accuracy between the three-dimensional model and the patient's airway, the clinician may, following automatic registration, utilize the systems and methods herein to perform an additional localized registration (or a registration update) of the airway at or near the identified target. In particular, and as described in more detail below, an ultrasound probe may be used to identify additional points of reference for use in updating and/or performing localized registration of the airway to the three-dimensional model.

The registration system of the present disclosure, for example, generally includes at least one sensor the location of which is tracked within an electromagnetic field. The location sensor may be incorporated into different types of tools, for example an ultrasound probe, and enables determination of the current location of the tool within a patient's airway by comparing the sensed location in space to locations within the three-dimensional model based on a mapping between location sensor signal values and corresponding locations with the three-dimensional model. The registration facilitates navigation of the sensor or a tool to a target location and/or manipulation of the sensor or tool relative to the target location. Navigation of the sensor or tool to the target location is more fully described in U.S. Patent Application Publication No. 2016/0000302.

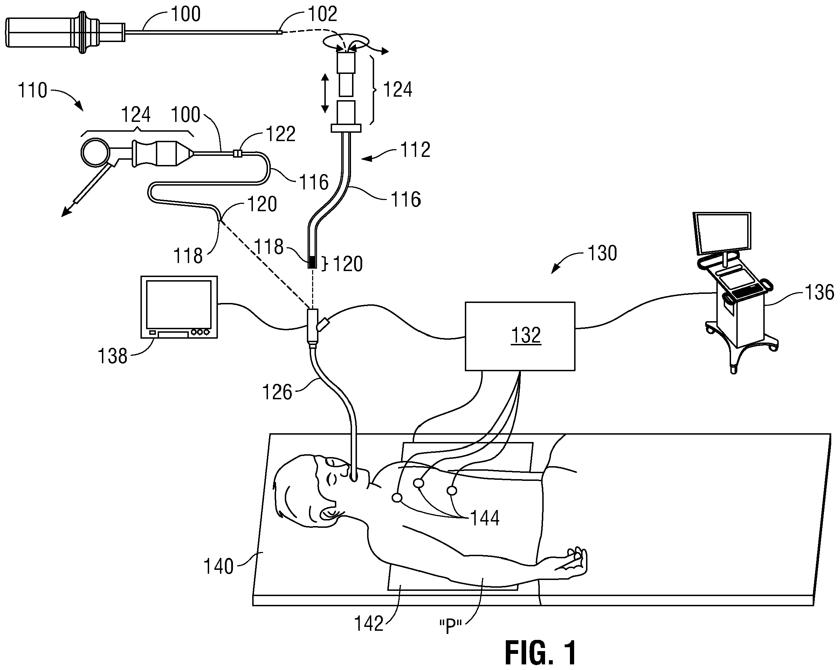

Referring now to FIG. 1, an electromagnetic navigation (EMN) system 130 configured for use with a catheter guide assembly 110, 112 is shown, in accordance with an example embodiment of the present disclosure. The EMN system 130 is configured to utilize CT imaging, magnetic resonance imaging (MRI), ultrasonic imaging, endoscopic imaging, fluoroscopic imaging, or another modality to create a roadmap of a patient's lungs. One such EMN system 130 is Medtronic Inc.'s ELECTROMAGNETIC NAVIGATION BRONCHOSCOPY® system. The EMN system 130 generally includes a bronchoscope 126 configured to receive one or more types of catheter guide assemblies 110, 112, monitoring equipment 138, an electromagnetic field generator 142, a tracking module 132, reference sensors 144, and a workstation 136. The workstation 136 includes software and/or hardware used to facilitate pathway planning, identification of a target, navigation to the target, and digitally marking a biopsy location. The target may be a lesion, tissue, a physical marker or structure, or any number of different locations within a body. FIG. 1 also depicts a patient "P" lying on the electromagnetic field generator 142, which is positioned upon an operating table 140. The locations of a number of reference sensors 144 placed on the patient "P" in the magnetic field generated by the electromagnetic field generator 142 can be determined by the tracking module 132. The EMN system 130 uses the reference sensors 144 to calculate a patient coordinate frame of reference.

Two example types of catheter guide assemblies 110, 112 usable with the EMN system 130 are depicted in FIG. 1. For a more detailed description of the example catheter guide assemblies 110, 112, reference is made to U.S. Patent Application Publication No. 2014/0046315, entitled MICROWAVE ABLATION CATHETER AND METHOD OF UTILIZING THE SAME, filed on March 15, 2013, the entire contents of which are hereby incorporated herein by reference. Each of the catheter guide assemblies 110, 112 includes a control handle 124 coupled to an extended working channel (EWC) 116 that is configured to receive a tool 100. The handle 124 can be manipulated by rotation and compression to steer distal end 118 of the EWC 116 and/or tool 100. The EWC 116 is sized for placement into the working channel of a bronchoscope 126. The EWC 116 may include an electromagnetic sensor 120 located on a distal end 118 of the EWC 116. The tool 100 may be any one of a variety of medical devices including, but not limited to, a locatable guide (LG), an ultrasound probe, a needle, a guide wire, a biopsy tool, a dilator, or an ablation device. In an embodiment, the tool 100 may also include its own electromagnetic sensor 120. In operation, a tool 100 including an electromagnetic sensor 120 is inserted into the EWC 116 and locked into position such that the electromagnetic sensor 120 extends a desired distance beyond a distal end 118 of the EWC 116. The electromagnetic sensor 120 works in conjunction with the tracking module 132 to enable tracking and navigation of the electromagnetic sensor 120, and thus of the distal end of the tool 100 and/or of the EWC 116, within the magnetic field generated by the electromagnetic field generator 142. In particular, the tracking module 132 receives location and/or orientation data corresponding to the electromagnetic sensor 120 that enables the electromagnetic sensor 120 to be tracked during navigation within a luminal network of the patient "P" toward a target site within the patient "P." Although the sensor 120 is described as being an electromagnetic sensor, the electromagnetic sensor 120 may be any suitable type of location sensor, such as, for example, a ring sensor, an optical sensor, a radiofrequency sensor, and/or the like. Additionally, the terms "luminal network," "airway," and "lungs" may be used interchangeably herein. Also, although the luminal network is described as an airway of the patient "P," this is by way of example only. Aspects of the present disclosure may also be applicable to other luminal networks, such as an intestinal network, and/or any other type of physiological structure within the patient "P."

As shown in FIG. 1, the electromagnetic field generator 142 is positioned beneath the patient "P." The electromagnetic field generator 142 and the reference sensors 144 are interconnected with the tracking module 132, which derives the location of each reference sensor 144 in six degrees of freedom. One or more of the reference sensors 144 are attached to the chest of the patient "P." The six degrees of freedom coordinates of the reference sensors 144 are sent to the workstation 136, which uses data collected by sensors 144 to calculate a patient coordinate frame of reference.

During procedure planning, the workstation 136 utilizes CT image data to generate and display the three-dimensional model of the airway of the patient "P," enables the identification of a target within the three-dimensional model (automatically, semi-automatically, or manually), and allows for the selection of a pathway through the airway of the patient "P" to the target. More specifically, the CT scans are processed and assembled into a three-dimensional volume, which is then utilized to generate the three-dimensional model of the airway of the patient "P." The three-dimensional model may be presented on a display monitor associated with the workstation 136, or in any other suitable fashion. Using the workstation 136, various slices of the three-dimensional volume, and views of the three-dimensional model may be presented and/or may be manipulated by a clinician to facilitate identification of a target and selection of a suitable pathway through the airway of the patient "P" to access the target. The three-dimensional model may also show marks of the locations where previous biopsies were performed, including the dates, times, and other identifying information regarding the tissue samples obtained. These marks may also be selected as the target to which a pathway can be planned. Once selected, the pathway is saved for use during the navigation procedure. During navigation, the system 130 enables tracking of the electromagnetic sensor 120 and/or the tool 100 as the electromagnetic sensor 120 and/or the tool 100 are advanced through the airway of the patient "P."

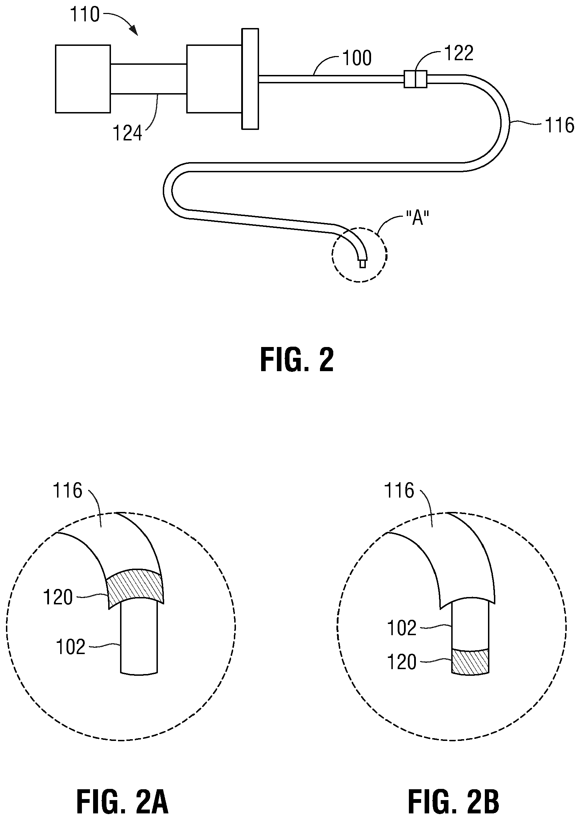

With additional reference to FIG. 2, an example catheter guide assembly 110 is shown, in accordance with an embodiment of the present disclosure. In addition to including the EWC 116 and the tool 100, the catheter guide assembly 110 includes the control handle 124, which enables advancement and steering of the distal end of the catheter guide assembly 110. Once inserted into the EWC 116, the tool 100 can be locked to the EWC 116 with a locking mechanism 122. The locking of tool 100 to the EWC 116 allows the tool 100 and the EWC 116 to travel together throughout a luminal network of the patient "P." The locking mechanism 122 may be a simple clip or luer lock, or the tool 100 may have a threaded configuration that allows it to threadably engage with and lock to the EWC 116. Examples of catheter guide assemblies usable with the present disclosure are currently marketed and sold by Medtronic Inc. under the name SUPERDIMENSION® Procedure Kits and EDGE™ Procedure Kits. For a more detailed description of catheter guide assemblies, reference is made to U.S. Patent Application Publication No. 2014/0046315 and U.S. Patent No. 7,233,820.

FIG. 2A is an enlarged view of a distal end of the catheter assembly 110 indicated by an encircled area "A" in FIG. 2. In this example, the EWC 116 including an electromagnetic sensor 120 is shown receiving a tool 100. In FIG. 2A, the tool 100 is an ultrasound (ultrasound) probe 102. In example embodiments, the ultrasound probe 102 is coupled to a distal end of the tool 100, while in an alternative embodiment, the ultrasound probe 102 comprises the entire tool 100. The ultrasound probe 102 includes at least one ultrasound transducer configured to transmit and receive ultrasound signals. FIG. 2B depicts a different example embodiment of the distal end of the catheter assembly 110. In this example embodiment, the ultrasound probe 102 includes an electromagnetic sensor 120, with the electromagnetic sensor 120 being embedded into the ultrasound probe 102. The electromagnetic sensor 120 may be positioned close to an ultrasound transducer of the ultrasound probe 102, to enable the location of the ultrasound probe 102 to be determined based on an electromagnetic field generated by the electromagnetic field generator 142. Although not shown in FIGS. 2A or 2B, in some embodiments, there are electromagnetic sensors 120 in both the EWC 116 and in the ultrasound probe 102. In some examples, the electromagnetic sensor 120 embedded into the ultrasound probe 102 includes two coils positioned at an angle with respect to each other (for example at a 90° angle or another angle), which can be used to sense a position of the probe with six degrees of freedom. In one example embodiment, the electromagnetic sensor 120 may be embedded into the ultrasound probe 102 at a non-zero angle (for example, at a 45° angle or another angle) with respect to the main axis of the ultrasound probe 102, and a roll angle of the ultrasound probe 102 may be determined based on the location of the electromagnetic sensor 120 of the EWC 116 and its spatial relationship with the electromagnetic sensor 120 embedded into the ultrasound probe 102. In this case, a local registration update can be performed as described herein for a target located some distance from the ultrasound probe 102.

For each configuration of the one or more electromagnetic sensors 120 in the EWC 116 and/or the ultrasound probe 102, one or more of the electromagnetic sensors 120 (for example, the electromagnetic sensor 120 of the EWC 116, the electromagnetic sensor 120 of the ultrasound probe 102, or both electromagnetic sensors 120) is used to track the location of the EWC 116 and/or the ultrasound probe 102 throughout the airway of the patient within the electromagnetic field generated by the electromagnetic field generator 142. For instance, the electromagnetic sensor 120 on the distal portion of the EWC 116 and/or the ultrasound probe 102 senses a signal (for example, a current and/or voltage signal) received based on the electromagnetic field produced by the electromagnetic generator 142, and provides the sensed signal to the tracking module 132 for its use in identifying the location and/or orientation of the electromagnetic sensor 120, the EWC 116, and/or the ultrasound probe 102 within the generated electromagnetic field. Thus, the location and/or orientation of the ultrasound probe 102 can be determined from the electromagnetic sensor 120 location. The electromagnetic sensor 120 is used to navigate the EWC 116 and/or ultrasound probe 102 through a luminal network of the patient "P." The ultrasound probe 102 is used to sense, locate, image, and/or identify, in real time, a target within or near the luminal network. In example embodiments, the ultrasound probe 102 is an endobronchial ultrasound (EBUS) or a radial endobronchial ultrasound (R-EBUS) probe. In various embodiments, a spatial relationship between the ultrasound probe 102 and the electromagnetic sensor 120 may be either fixed or variable. In embodiments where the spatial relationship between the ultrasound probe 102 and the electromagnetic sensor 120 is fixed (for example, mechanically fixed), a value of the spatial relationship may be measured before an EMN procedure is conducted and the value may be used during the EMN procedure to determine a location of the ultrasound probe 102 based on a determined location of the electromagnetic sensor 120. In embodiments where the spatial relationship between the ultrasound probe 102 and the electromagnetic sensor 120 is variable, the value of the spatial relationship may be determined before and/or during an EMN procedure.

In an example embodiment where the ultrasound probe 102 is an R-EBUS probe, the distance the ultrasound probe 102 extends distally past the EWC 116 may be determined. This can be accomplished by using markers on the shaft of the ultrasound probe 102, or a locking mechanism, such as the locking mechanism 122, to fix the distance. Alternatively, in one example embodiment, both the EWC 116 and the ultrasound probe 102 contain separate electromagnetic sensors 120. For example, in order to fit into a catheter, a needle-like electromagnetic sensor 120 wrapped around a mu-metal core may be embedded into the R-EBUS probe. In this example embodiment, a spatial relationship between the ultrasound probe 102 and the electromagnetic sensor 120 of the EWC 116 can be determined based on signals from the respective electromagnetic sensors 120 of the EWC 116 and the ultrasound probe 102. In this manner, the location of the ultrasound probe 102 relative to the EWC 116, and thus the distance the ultrasound probe 102 extends distally past the EWC 116 can also be determined.

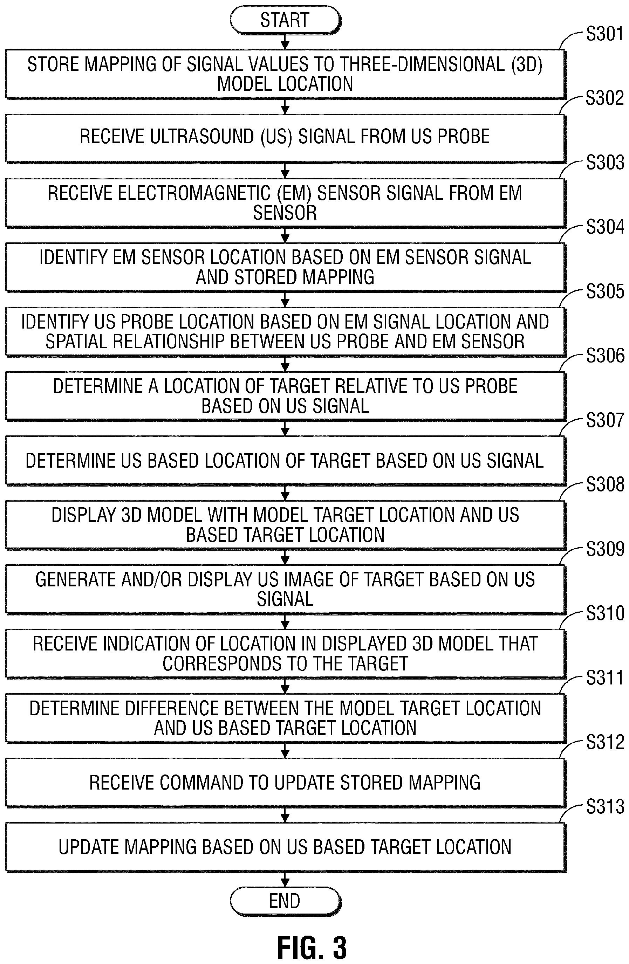

Having described the example EMN system 130, reference will now be made to FIG. 3, which illustrates an example method 300 for electromagnetic navigation registration that the example EMN system 130 may implement. At S301 a mapping is stored in a memory, such as, for example, a memory of the tracking module 132, the workstation 136 or of another component of the system 130. In general, the mapping is utilized during an EMN procedure to determine, based on a value of a signal sensed by the electromagnetic sensor 120 during the EMN procedure, the location of the electromagnetic sensor 120 within a volume of the electromagnetic field generated by the electromagnetic field generator 142, and within the airway of the patient "P." In particular, the mapping associates electromagnetic field-based signal values with corresponding locations within a three-dimensional model of a luminal network of the patient "P." With the patient "P" positioned above the electromagnetic field generator 142, the mapping can be used by extension to associate the electromagnetic field-based signal values with corresponding locations within the actual luminal network of the patient "P." The electromagnetic field-based signal values are signals (such as, for example, magnitude and/or frequency components of current signals and/or voltage signals) that may be sensed by way of the electromagnetic sensor 120 based on an electromagnetic field generated by the electromagnetic field generator 142.

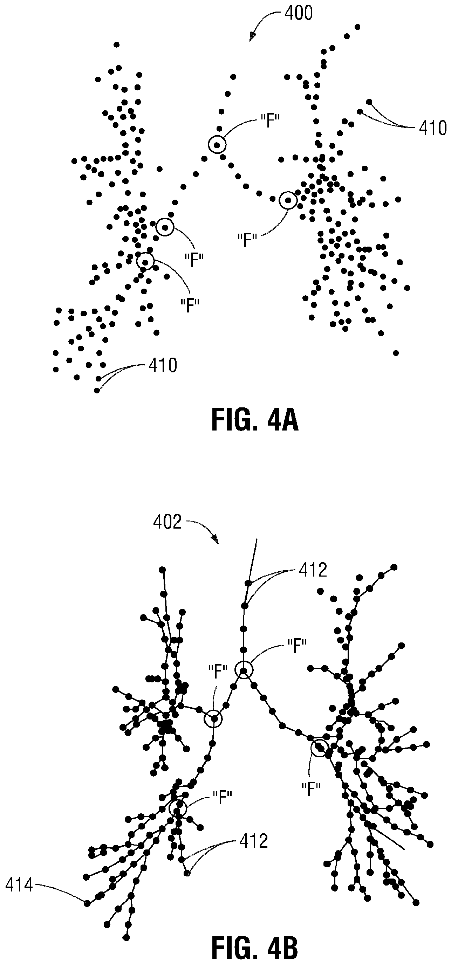

In one example embodiment, the mapping may be generated prior to S301, based on a survey and an initial registration procedure, during which spatial locations of the electromagnetic sensor 120 are mapped to corresponding spatial structure of the luminal network of the patient "P." In some examples, the mapping and a pathway plan to a target in the patient "P" may be imported into navigation and procedure software stored on a computer such as the workstation 136 of FIG. 1. Before continuing to describe the method 300, reference will briefly be made to FIGS. 4A and 4B, to describe an example of the initial registration of the electromagnetic sensor 120 location in space to the spatial structure of the lungs. FIG. 4A illustrates a body space model (BS model) 400 of an airway of the patient "P" generated during an initial electromagnetic navigation registration procedure. The BS model 400 contains multiple survey points 410 generated during a survey procedure by sampling signals sensed by the electromagnetic sensor 120 as it is navigated through various branches of the airway of the patient "P." In particular, at each of the survey points 410, which corresponds to a particular location within the airway of the patient "P," the system 130 collects a signal value sensed by the electromagnetic sensor 120 based on the electromagnetic field generated by the electromagnetic field generator 142. In this manner, each of the survey points 410 represents an entry of the stored mapping and associates a particular electromagnetic field-based signal value with a corresponding location within a three-dimensional model 402 (described below) of the luminal network of the patient "P." Certain survey points 410 may be designated and/or selected as fiducial points "F" within the BS model 400. For example, prominent locations and/or features that are less prone to being mistaken for a different location and/or feature by a clinician (for instance, survey points 410 located at defined intersections in the airway where airway branches branch apart from each other) may be designated as fiducial points "F." Following registration of the airway of the patient "P," the workstation 136 retrieves the survey points 410 and generates a BS model 400 of the patient's airway based on the plurality of survey points 410.

FIG. 4B illustrates a three-dimensional model 402 of the airway of the patient "P" generated from a CT scan. The three-dimensional model 402 includes a plurality of reference points 412 collected during a CT scan of the patient's airway. The reference points 412, when mapped together, form a variety of pathways through the branches of the patient's airway. The three-dimensional model 402 also includes fiducial points "F" which can be mapped to the same fiducial points "F" determined in the BS model and serve as the main reference points 412. Additionally, a target can be identified from the CT scan images and a modeled location of the target 414 within the three-dimensional model 402 can be determined and represented in the stored mapping. For example, the locations within the three-dimensional model may include the modeled location of the target, and the mapping stored at S301 may associate one or more of the electromagnetic field-based signal values with the modeled location of the target. Accordingly, the workstation 136 can use the three-dimensional mapping 402 to determine and generate a pre-planned pathway to reach the modeled target location 414. During registration of the three-dimensional model 402 to the patient's airway, the survey points 410 of the BS model 400 are mapped and/or interpolated to corresponding reference points 412 of the three-dimensional model 402, for example, by executing a Thin Plate Splines (TPS)-based algorithm. Thus, the mapping can be utilized to determine the location of the electromagnetic sensor 120 within the patient's airway during an EMN procedure.

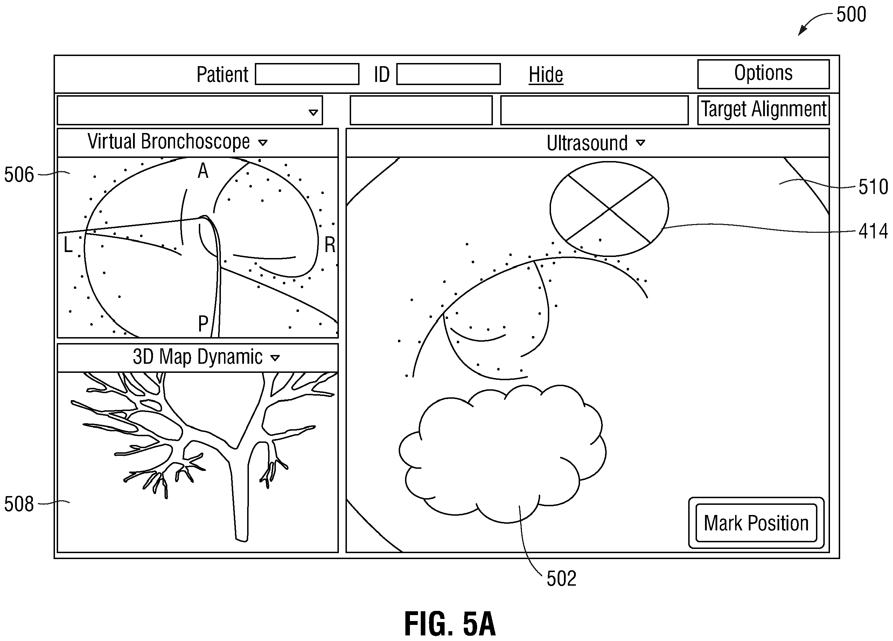

However, because, in some cases, the survey points 410 may be limited to the relatively few first generations of the patient's airway and the patient's airway is flexible, there can be differences between the three-dimensional model 402 and the structure of the airway of the patient "P" during a subsequent EMN procedure. These differences may be referred to as CT-to-body divergence, which can result in registration errors and may lead to errors in locating targets within patients. As described in more detail below, these errors can be mitigated or effectively eliminated by adding additional survey points 410 that correspond to additional reference points 412 proximal to the target itself. For example, in general, an ultrasound probe 102 can be used to identify an ultrasound-based location of the target 502 (FIG. 5A) that is expected to correspond to the modeled location of the target 414 in the three-dimensional model 402, and the stored mapping may be updated based on the ultrasound-based location of the target 502. A more detailed explanation of the registration and pathway planning system is described in U.S. Patent Application Publication Nos. 2014/0281961, 2014/0270441, and 2014/0282216.

As described above in the context of FIG. 1, during an EMN procedure, the electromagnetic sensor 120 and the ultrasound probe 102 are inserted into the patient's airway via a natural orifice or an incision. Referring now back to FIG. 3, at S302 an ultrasound signal is received from the ultrasound probe 102 while the ultrasound probe 102 is located within the airway of the patient "P," for example proximal to the target.

The electromagnetic field generator 142 generates an electromagnetic field that overlaps with the volume occupied by the airway of the patient "P." At S303, an electromagnetic sensor signal is received from the electromagnetic sensor 120, while the electromagnetic sensor 120 is located within the airway of the patient "P," for example proximal to the target. The received signal is based on the electromagnetic field generated by the electromagnetic field generator 142. In general, the receiving of the ultrasound signal at S302 occurs while the ultrasound probe 102 and the electromagnetic sensor 120 remain substantially stationary within the patient "P", so as to enable the location of the ultrasound probe 102 and/or the ultrasound-based target location 414 to be determined based on the determined location of the electromagnetic sensor 120. For example, the ultrasound probe 102 and the electromagnetic sensor 120 may remain positioned in their respective locations in the patient during the receiving of the ultrasound signal and electromagnetic sensor signal at S302 and S303, respectively.

At S304, a location within the three-dimensional model that corresponds to the received value of the electromagnetic sensor signal (also referred to herein as an "electromagnetic sensor location") is identified based on a value of the electromagnetic sensor signal received at S303 and based on the mapping stored at S301. For example, the electromagnetic sensor location may be determined by performing a look-up in the mapping, based on the received value of the electromagnetic field-based signal, to identify which location within the three-dimensional model of the luminal network of the patient "P" is associated with the received electromagnetic field-based signal value.

At S305, a location within the three-dimensional model that corresponds to the ultrasound signal received at S302 (referred to herein as an "ultrasound probe location") is identified based on the electromagnetic sensor location identified at S304 and based on a spatial relationship between the ultrasound probe 102 and the electromagnetic sensor 120. For example, as mentioned above, in various embodiments, a spatial relationship between the ultrasound probe 102 and the electromagnetic sensor 120 may be either fixed or variable. In embodiments where the spatial relationship between the ultrasound probe 102 and the electromagnetic sensor 120 is fixed (for example, mechanically fixed), the value of the spatial relationship may be determined and/or measured before the EMN procedure is conducted. In embodiments where the spatial relationship between the ultrasound probe 102 and the electromagnetic sensor 120 is variable, the value of the spatial relationship may be determined in the manner described above, before and/or during an EMN procedure. The spatial relationship value may be used at S305, during the EMN procedure for example, to determine the location of the ultrasound probe 102 based on the location of the electromagnetic sensor 120 determined at S304.

At S306, a location of the target relative to the ultrasound probe 102 is determined based on the ultrasound signal received at S302. In particular, the ultrasound probe 102 may transmit and receive ultrasound waves by which an ultrasound image of the target may be generated. Based on the generated ultrasound image of the target, the location of the target relative to the ultrasound probe 102 may be determined at S306.

At S307, an ultrasound-based location of the target 502, relative to the three-dimensional model 402, is determined based on the ultrasound signal received at S302. For example, the ultrasound-based location of the target 502 may be determined based on the location of the target relative to the ultrasound probe 102 determined at S306, the electromagnetic sensor location identified at S304 and/or the ultrasound probe location identified at S305. In particular, with the electromagnetic sensor location identified at S304 relative to the three-dimensional model having been identified, the ultrasound-based location of the target 502 may be computed taking into account the ultrasound probe location relative to the three-dimensional model (and/or the spatial relationship between the ultrasound probe 102 and the electromagnetic sensor 120) and the location of the target relative to the ultrasound probe 102 determined at S306.

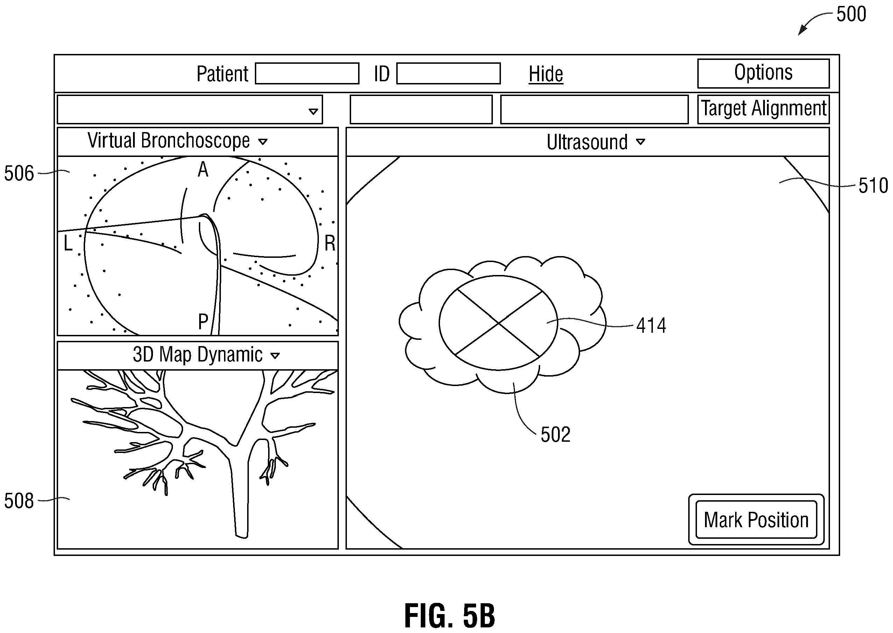

At S308, at least a portion of the three-dimensional model 402 (or a graphical rendering thereof) is displayed via a graphical user interface (GUI), such as a GUI of the monitoring equipment 138 or the workstation 136, based on the electromagnetic sensor location identified at S304 and/or based on the ultrasound probe location identified at S305. Also displayed via the GUI are an indication of the modeled location of the target 414 relative to at least the displayed portion of the three-dimensional model 402, and an indication of the ultrasound-based location of the target 502 relative to at least the portion of the three-dimensional model 402. Before continuing to describe the procedure 300, reference will briefly be made to FIGS. 5A and 5B to describe an example GUI that may be employed at S308.

FIGS. 5A and 5B show views of a user interface (for example, a GUI) 500 that enables a clinician to navigate an instrument (for example, the ultrasound probe 102) to a target within the patient "P." The user interface 500 includes a number of windows with different views. In particular, user interface 500 includes a virtual bronchoscope view 506, a three-dimensional map dynamic view 508, and an ultrasound view 510. Although not depicted in the user interface 500, a number of different views are also envisioned. For example, the user interface 500 may also include different CT views and/or a live bronchoscope view. Additionally, the arrangement of the views is not limited to the arrangement depicted in FIGS. 5A or 5B.

The virtual bronchoscope view 506 presents the clinician with a three-dimensional rendering of the walls of the patient's airways generated from the CT images which form the three-dimensional model 402, as shown, for example, in FIG. 5A.

The three-dimensional map dynamic view 508 presents a dynamic view of the three-dimensional model 402 of the patient's airways. In particular, the three-dimensional map dynamic view 508 presents the clinician with a navigation pathway providing an indication of the direction along which the clinician will need to move the ultrasound probe 102 to reach the modeled target location 414. The three-dimensional map dynamic view 508 may also present a live view of the location of the ultrasound probe 102, for example, as ascertained based on a determined location of the electromagnetic sensor 120, to assist the clinician in navigating the ultrasound probe 102 towards the modeled target location 414.

The ultrasound view 510 presents the clinician with a real-time ultrasound image (for example, of the target and/or the surrounding area within the airway of the patient "P") generated based on an ultrasound signal received from the ultrasound probe 102. The ultrasound view 510 enables the clinician to visually observe the patient's airways in real-time as the ultrasound probe 102 is navigated through the patient's airways toward the target. Using the virtual bronchoscope view 506 and the three-dimensional map dynamic view 508, the clinician navigates the ultrasound probe 102 towards the expected location of modeled target location 414. As the ultrasound probe 102 nears the target, an indication of the ultrasound-based location of the target 502 is displayed (for example, as an overlay) via the ultrasound view 510. Also displayed via the ultrasound view 510 is an indication of the modeled target location 414, which may be determined based at least in part on the three-dimensional model 402 (for example, based on a previously performed CT scan) and/or the mapping stored at S301. In this manner, a combined view of an indication of the modeled location of the target 414, relative to at least a portion of the three-dimensional model, and an indication of the ultrasound-based location of the target 502, relative to at least the portion of the three-dimensional model, may be simultaneously displayed via the ultrasound view 510, enabling a difference between the two locations to be ascertained, by way of a clinician's observation and/or by way of automatic techniques, such as one or more known image processing algorithms, for example, using distinct contrast of the ultrasound-based target image. As described above, the ultrasound-based location of the target 502 determined based at least in part on the signal from the ultrasound probe 102 may differ from the modeled target location 414 as determined by the three-dimensional model 402 and/or the mapping as a result of CT-to-body divergence. An example of a difference in the modeled target location 414 and the ultrasound-based target location 502 is depicted in FIG. 4A.

Referring back to FIG. 3, at S309, an ultrasound image of the target in the patient "P" is generated and/or displayed (for example, as described above in connection with FIGS. 5A and 5B) based on the signal received from the ultrasound probe at S302.

With continued reference to FIGS. 3, 5A, and 5B, at S310 an indication of a location within the displayed portion of the three-dimensional model that corresponds to the target is received by way of the user interface 500, and the ultrasound-based location of the target determined at S307 may be based on the received indication of the location. In particular, once the ultrasound probe 102 is positioned in proximity to the target and the ultrasound-based location of the target 502 is displayed via the ultrasound view 510, the clinician can identify the target by way of the user interface 500 or another input device associated therewith (for example, by using a mouse to click in the center of the target). For example, the user may provide, by way of the user interface 500, an indication of a location within the displayed portion of the three-dimensional model in the ultrasound view 510 that corresponds to the target (for example, a center of the ultrasound-based target location 502). The clinician can, for instance, either touch the display at the indicated location if the display is a touchscreen display, or the clinician can indicate the location using a computer cursor, or another user input device. As described below, the ultrasound-based location of the target 502 may be determined based on the location that is indicated by the user as corresponding to the target. Once the ultrasound-based target location 502 is identified, the workstation 136 can determine an updated location of the target relative to the three-dimensional model 402 based on the ultrasound-based target location 502. The updated location of the target can then be used as an additional survey point 410 that corresponds to the modeled target location 414 in three-dimensional model 402. If there is a difference in the ultrasound-based location of the target 502 and the modeled target location 414, workstation 136 can update the registration of the three-dimensional model 402 to the BS model 400. As shown in the ultrasound view 510 of FIG. 5B, once the registration has been updated, the ultrasound-based target location 502 will match the modeled target location 414.

In one example, the locations within the three-dimensional model include the modeled location of the target 414, and the mapping associates one or more of the electromagnetic field based signal values with the modeled location of the target 414. At S311, a difference between the modeled location of the target 414 (with respect to the three-dimensional model) and the ultrasound-based target location 502 (with respect to the three-dimensional model) is determined based on the ultrasound probe location identified at S305 and/or the electromagnetic sensor location identified at S304. In some example embodiments, the difference between the modeled location of the target 414 and the ultrasound-based location of the target 502 is determined at S311 by executing one or more known image processing algorithms based on a combined view of an indication of the modeled location of the target 414 and the indication of the ultrasound-based location of the target 502.

At S312, a command to update the mapping is received by way of the user interface 500 or another user input device. Alternatively, a clinician may avoid inputting the command to update the mapping, to leave the mapping unchanged, for example, if the difference between the modeled target location 414 and the ultrasound-based target location 502 is minimal.

At S313, at least a portion of the mapping stored at S301 is updated based on the ultrasound-based target location 502. In one example, the updating at S313 is performed in response to the receiving of the command at S312. In another example, the updating at S313 is automatically performed, without requiring input from the user, for example, based on an automatically determined difference between the modeled target location 414 and the ultrasound-based target location 502. The updating of the mapping, in some embodiments, includes modifying the mapping to associate a different one or more of the electromagnetic field-based signal values (for example, a value of the electromagnetic field-based signal received at S303) with the modeled location of the target 414. In this manner, the modeled target location 414 is corrected based on the ultrasound-based target location 502, which in some cases may be more accurate than the original modeled target location 502 before the updating at S313.

In another example embodiment, a mathematical interpolation algorithm is executed on the mapping entries, based on the modeled target location 414 that was updated at S313 and/or based on the difference between the modeled target location 414 and the ultrasound-based target location 502 determined at S311. The employed interpolation algorithm may include a thin plate splines (TPS) algorithm or any other suitable interpolation algorithm. The interpolation algorithm may be based on one or more pairs of additional pairs of points, each pair including a point obtained from the electromagnetic modality (by way of the electromagnetic sensor 120) and a corresponding point obtained from the ultrasound modality (by way of the ultrasound probe 102. One such pair may be based on the ultrasound-based target location determined at S307 and the modeled target location before being updated. Additional pairs of points may be obtained or generated, for example, at other locations (for example, where the airway branches into multiple paths) within the patient's airway, and, based on the pairs of points, a global interpolation function can be generated by which the mapping can be updated at S313. For instance, the updating of the mapping at S313 may further include modifying the mapping to change which of multiple electromagnetic field-based signal values are associated with which of multiple locations within the three-dimensional model, respectively, based on a result of the executing of the interpolation algorithm. In this manner, not only can the target location itself be updated based on the ultrasound-based location 502, but other portions of the mapping may also be updated based on the ultrasound-based location 502. This may improve the accuracy of the mapping with respect to the target location itself (for example, for targets located in peripheral areas of the lung) as well as locations proximal to the target location. In some cases, for example, depending on the locations of the pairs of points utilized, the mapping may be updated in a region local to the target but other portions of the mapping may remain substantially unchanged. Once the mapping has been updated at S313, the ultrasound probe 102 may be removed from the EWC 116, which remains within the patient "P," and the clinician may insert a different tool into the EWC 116, to perform a procedure utilizing the updated and improved mapping by way of the electromagnetic sensor 120 of the EWC 116.

As can be appreciated in view of the present disclosure, ultrasound imaging can provide greater resolution than CT imaging when at the very granular level of a location where a biopsy is desired, for example. When in the periphery of the lung, where the airways are small and the images tend to breakdown, CT image data may be less reliable for accurate EMN purposes. Real-time ultrasound using the ultrasound probe 102 can provide more accurate information as to where the clinician has placed a tool or navigated to and can increase the accuracy of biopsy, treatment, and/or post-treatment assessment. The system 130 utilizing the ultrasound probe 102 can generate data in the form of ultrasound imaging data that can be incorporated into the existing navigation pathway. This data may be in the form of a side-by-side image that can be manually compared by a trained clinician to confirm their location or to achieve a more exacting location where EMN achieved only an approximate location near a target, as described in more detail above with reference to FIGS. 5A and 5B. The ultrasound data obtained from the ultrasound probe 102 can be used to confirm registration of the patient to the three-dimensional model 402, perform re-registration, or perform a local registration in an effort to provide greater clarity of the tissue at the desired location and confirm that the clinician has achieved the desired location in the patient.

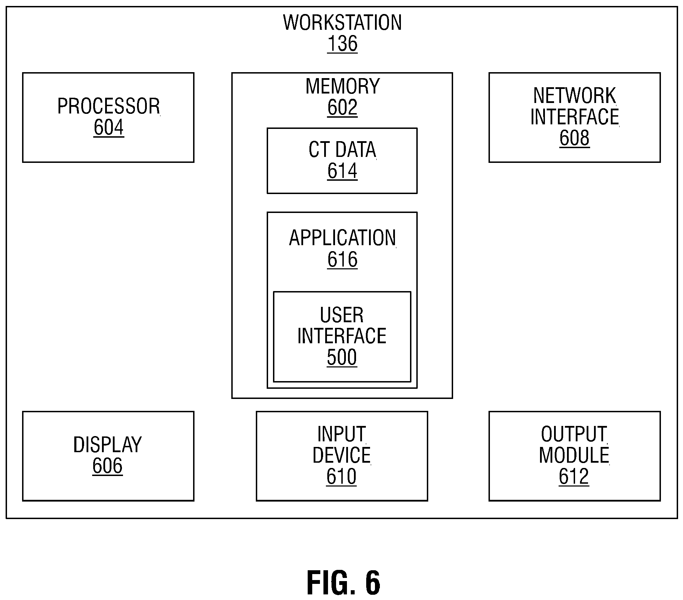

Turning now to FIG. 6, there is shown a system diagram having components that may be included in the workstation 136. Alternatively, the components shown in FIG. 6 may be included in the tracking module 132, the monitoring equipment 138, and/or in another device. The workstation 136 may include a memory 602, a processor 604, a display 606, network interface 608, input device 610, and/or output module 612.

The memory 602 includes non-transitory computer-readable storage media for storing data and/or software that is executable by the processor 604 and which controls the operation of the workstation 136. In an example embodiment, the memory 602 may include one or more solid-state storage devices such as flash memory chips. Alternatively, or in addition to the one or more solid-state storage devices, the memory 602 may include one or more mass storage devices connected to the processor 604 through a mass storage controller (not shown in FIG. 6) and a communications bus (not shown in FIG. 6). Although the description of computer-readable media contained herein refers to a solid-state storage, it should be appreciated by those skilled in the art that computer-readable storage media can be any available media that can be accessed by the processor 604. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by workstation 136.

The memory 602 may store an application (for example an application that provides the GUI 500) and/or CT data 614. In particular, the application may, when executed by the processor 604, cause the display 606 to present the user interface 500. The network interface 608 may be configured to connect to a network such as a local area network (LAN) consisting of a wired network and/or a wireless network, a wide area network (WAN), a wireless mobile network, a Bluetooth network, and/or the Internet. The input device 610 may be any device by means of which a user may interact with the workstation 136, such as, for example, a mouse, a keyboard, a foot pedal, a touch screen, and/or a voice interface. The output module 612 may include any connectivity port or bus, such as, for example, a parallel port, a serial port, a universal serial bus (USB), or any other similar connectivity port known to those skilled in the art.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

The invention may be described by reference to the following numbered paragraphs:-
1. A method for electromagnetic navigation registration, comprising:
   storing, in a memory, a mapping that associates electromagnetic field-based signal values with corresponding locations within a three-dimensional model of a luminal network;
   receiving an ultrasound signal from an ultrasound probe;
   determining, based on the ultrasound signal, an ultrasound-based location of a target in a patient relative to the three-dimensional model; and
   updating at least a portion of the mapping based on the ultrasound-based location of the target.
2. The method according to paragraph 1, further comprising:
   receiving an electromagnetic sensor signal from an electromagnetic sensor;
   identifying, based on a value of the electromagnetic sensor signal and the mapping, an electromagnetic sensor location within the three-dimensional model that corresponds to the value of the electromagnetic sensor signal; and
   identifying an ultrasound probe location within the three-dimensional model that corresponds to the ultrasound signal, based on the electromagnetic sensor location and a spatial relationship between the ultrasound probe and the electromagnetic sensor.
3. The method according to paragraph 2, further comprising:
   determining, based on the ultrasound signal, a location of the target relative to the ultrasound probe;
   wherein the ultrasound-based location of the target is determined based on (i) the location of the target relative to the ultrasound probe and (ii) at least one of the electromagnetic sensor location or the ultrasound probe location.
4. The method according to paragraph 2, wherein the spatial relationship between the ultrasound probe and the electromagnetic sensor is fixed.
5. The method according to paragraph 2, wherein the spatial relationship between the ultrasound probe and the electromagnetic sensor is variable.
6. The method according to paragraph 2, wherein the receiving of the ultrasound signal occurs while the ultrasound probe and the electromagnetic sensor are positioned in respective locations in the patient, and the receiving of the electromagnetic sensor signal occurs while the ultrasound probe and the electromagnetic sensor are positioned in the respective locations in the patient.
7. The method according to paragraph 2, wherein the locations within the three-dimensional model include a modeled location of the target, and the mapping associates one or more of the electromagnetic field-based signal values with the modeled location of the target, and the method further comprises:
   determining a difference between the modeled location of the target and the ultrasound-based location of the target, based on at least one of the ultrasound probe location or the electromagnetic sensor location.
8. The method according to paragraph 2, further comprising:
   displaying, via a graphical user interface:
      at least a portion of the three-dimensional model, based on at least one of the electromagnetic sensor location or the ultrasound probe location,
      an indication of the modeled location of the target relative to at least the portion of the three-dimensional model, and
      an indication of the ultrasound-based location of the target relative to at least the portion of the three-dimensional model.
9. The method according to paragraph 8, further comprising:
   generating an image of the target based on the ultrasound signal,
   wherein the indication of the ultrasound-based location of the target is the image of the target.
10. The method according to paragraph 8, wherein the displaying includes simultaneously displaying a combined view of:
   the indication of the modeled location of the target relative to at least the portion of the three-dimensional model, and
   the indication of the ultrasound-based location of the target relative to at least the portion of the three-dimensional model.
11. The method according to paragraph 10, wherein the locations within the three-dimensional model include a modeled location of the target, and the mapping associates one or more of the electromagnetic field-based signal values with the modeled location of the target, and the method further comprises:
   determining a difference between the modeled location of the target and the ultrasound-based location of the target, based on image processing of the combined view of the indication of the modeled location of the target and the indication of the ultrasound-based location of the target.
12. The method according to paragraph 11, wherein the updating at least the portion of the mapping is automatically performed based on the difference between the modeled location of the target and the ultrasound-based location of the target.
13. The method according to paragraph 8, further comprising:
   receiving, by way of a user interface, an indication of a location within at least the displayed portion of the three-dimensional model that corresponds to the target,
   wherein the determining the ultrasound-based location of the target is based on the indication of the location that corresponds to the target.
14. The method according to paragraph 13, further comprising:
   receiving, by way of the user interface, a command to update the mapping,
   wherein the updating at least the portion of the mapping is performed in response to the receiving of the command.
15. The method according to paragraph 1, wherein the locations within the three-dimensional model include a modeled location of the target, and the mapping associates one or more of the electromagnetic field-based signal values with the modeled location of the target, and the method further comprises:
   determining a difference between the modeled location of the target and the ultrasound-based location of the target.
16. The method according to paragraph 15, wherein the updating at least the portion of the mapping is based on the difference between the modeled location of the target and the ultrasound-based location of the target.
17. The method according to paragraph 15, wherein the updating at least the portion of the mapping includes modifying the mapping to associate a different one or more of the electromagnetic field-based signal values with the modeled location of the target.
18. The method according to paragraph 15, further comprising:
   executing an interpolation algorithm based on the difference between the modeled location of the target and the ultrasound-based location of the target,
   wherein the updating at least the portion of the mapping further includes modifying the mapping to associate a plurality of the electromagnetic field-based signal values with a plurality of the locations within the three-dimensional model, respectively, based on a result of the executing of the interpolation algorithm.
19. The method according to paragraph 1, wherein the luminal network is an airway of the patient.
20. A method for electromagnetic navigation registration, comprising:
   receiving a signal from an ultrasound probe;
   generating, based on the signal received from the ultrasound probe, an ultrasound image of a target in a patient;
   determining, based on the ultrasound image, a location of the target relative to the ultrasound probe;
   receiving a signal from an electromagnetic sensor;
   determining, based on the signal received from the electromagnetic sensor, a location of the electromagnetic sensor relative to a three-dimensional model of a luminal network;
   determining an ultrasound-based location of the target relative to the three-dimensional model, based on the location of the target relative to the ultrasound probe, the location of the electromagnetic sensor relative to the three-dimensional model, and a spatial relationship between the ultrasound probe and the electromagnetic sensor; and
   updating, based on the ultrasound-based location of the target, a mapping that associates electromagnetic field-based signal values with corresponding locations within the three-dimensional model.

## Claims

1. A method for electromagnetic navigation registration, comprising:
storing, in a memory, a mapping that associates electromagnetic field-based signal values with corresponding locations within a three-dimensional model of a luminal network;
receiving an ultrasound signal from an ultrasound probe;
determining, based on the ultrasound signal, an ultrasound-based location of a target in a patient relative to the three-dimensional model; and
updating at least a portion of the mapping based on the ultrasound-based location of the target.

2. The method according to claim 1, further comprising:
receiving an electromagnetic sensor signal from an electromagnetic sensor;
identifying, based on a value of the electromagnetic sensor signal and the mapping, an electromagnetic sensor location within the three-dimensional model that corresponds to the value of the electromagnetic sensor signal; and
identifying an ultrasound probe location within the three-dimensional model that corresponds to the ultrasound signal, based on the electromagnetic sensor location and a spatial relationship between the ultrasound probe and the electromagnetic sensor.

3. The method according to claim 2, further comprising:
determining, based on the ultrasound signal, a location of the target relative to the ultrasound probe;
wherein the ultrasound-based location of the target is determined based on (i) the location of the target relative to the ultrasound probe and (ii) at least one of the electromagnetic sensor location or the ultrasound probe location.

4. The method according to claim 2, wherein the spatial relationship between the ultrasound probe and the electromagnetic sensor is fixed or alternatively , wherein the spatial relationship between the ultrasound probe and the electromagnetic sensor is variable.

5. The method according to claim 2, wherein the receiving of the ultrasound signal occurs while the ultrasound probe and the electromagnetic sensor are positioned in respective locations in the patient, and the receiving of the electromagnetic sensor signal occurs while the ultrasound probe and the electromagnetic sensor are positioned in the respective locations in the patient.

6. The method according to claim 2, wherein the locations within the three-dimensional model include a modeled location of the target, and the mapping associates one or more of the electromagnetic field-based signal values with the modeled location of the target, and the method further comprises:
determining a difference between the modeled location of the target and the ultrasound-based location of the target, based on at least one of the ultrasound probe location or the electromagnetic sensor location.

7. The method according to claim 2, further comprising:
displaying, via a graphical user interface:
at least a portion of the three-dimensional model, based on at least one of the electromagnetic sensor location or the ultrasound probe location,
an indication of the modeled location of the target relative to at least the portion of the three-dimensional model, and
an indication of the ultrasound-based location of the target relative to at least the portion of the three-dimensional model.

8. The method according to claim 7, further comprising:
generating an image of the target based on the ultrasound signal,
wherein the indication of the ultrasound-based location of the target is the image of the target.

9. The method according to claim 7, wherein the displaying includes simultaneously displaying a combined view of:
the indication of the modeled location of the target relative to at least the portion of the three-dimensional model, and
the indication of the ultrasound-based location of the target relative to at least the portion of the three-dimensional model.

10. The method according to claim 9, wherein the locations within the three-dimensional model include a modeled location of the target, and the mapping associates one or more of the electromagnetic field-based signal values with the modeled location of the target, and the method further comprises:
determining a difference between the modeled location of the target and the ultrasound-based location of the target, based on image processing of the combined view of the indication of the modeled location of the target and the indication of the ultrasound-based location of the target, preferably wherein the updating at least the portion of the mapping is automatically performed based on the difference between the modeled location of the target and the ultrasound-based location of the target.

11. The method according to claim 7, further comprising:
receiving, by way of a user interface, an indication of a location within at least the displayed portion of the three-dimensional model that corresponds to the target,
wherein the determining the ultrasound-based location of the target is based on the indication of the location that corresponds to the target preferably further comprising:
receiving, by way of the user interface, a command to update the mapping,
wherein the updating at least the portion of the mapping is performed in response to the receiving of the command.

12. The method according to claim 1, wherein the locations within the three-dimensional model include a modeled location of the target, and the mapping associates one or more of the electromagnetic field-based signal values with the modeled location of the target, and the method further comprises:
determining a difference between the modeled location of the target and the ultrasound-based location of the target, preferably, wherein the updating at least the portion of the mapping is based on the difference between the modeled location of the target and the ultrasound-based location of the target.

13. The method according to claim 12, wherein the updating at least the portion of the mapping includes modifying the mapping to associate a different one or more of the electromagnetic field-based signal values with the modeled location of the target.

14. The method according to claim 12, further comprising:
executing an interpolation algorithm based on the difference between the modeled location of the target and the ultrasound-based location of the target,
wherein the updating at least the portion of the mapping further includes modifying the mapping to associate a plurality of the electromagnetic field-based signal values with a plurality of the locations within the three-dimensional model, respectively, based on a result of the executing of the interpolation algorithm.

15. A method for electromagnetic navigation registration, comprising:
receiving a signal from an ultrasound probe;
generating, based on the signal received from the ultrasound probe, an ultrasound image of a target in a patient;
determining, based on the ultrasound image, a location of the target relative to the ultrasound probe;
receiving a signal from an electromagnetic sensor;
determining, based on the signal received from the electromagnetic sensor, a location of the electromagnetic sensor relative to a three-dimensional model of a luminal network;
determining an ultrasound-based location of the target relative to the three-dimensional model, based on the location of the target relative to the ultrasound probe, the location of the electromagnetic sensor relative to the three-dimensional model, and a spatial relationship between the ultrasound probe and the electromagnetic sensor; and
updating, based on the ultrasound-based location of the target, a mapping that associates electromagnetic field-based signal values with corresponding locations within the three-dimensional model.
